(19) Europäisches Patentamt / European Patent Office / Office européen des brevets

(11) **EP 2 391 545 B1**

(12) **EUROPEAN PATENT SPECIFICATION**

(45) Date of publication and mention
of the grant of the patent:
**19.04.2017 Bulletin 2017/16**

(51) Int Cl.:
**B65B 3/32** *(2006.01)*       **B65B 43/59** *(2006.01)*
**A61K 8/03** *(2006.01)*       **A61Q 5/02** *(2006.01)*
**B65D 77/08** *(2006.01)*

(21) Application number: **10703568.5**

(22) Date of filing: **28.01.2010**

(86) International application number:
**PCT/US2010/022360**

(87) International publication number:
**WO 2010/088353 (05.08.2010 Gazette 2010/31)**

(54) **PERSONAL CARE ARTICLE FOR SEQUENTIALLY DISPENSING COMPOSITIONS WITH VARIABLE CONCENTRATIONS OF HYDROPHOBIC BENEFIT MATERIALS**

KÖRPERPFLEGEARTIKEL FÜR AUFEINANDERFOLGENDE AUSGABE VON ZUSAMMENSETZUNGEN MIT VERSCHIEDENEN KONZENTRATIONEN HYDROPHOBER PFLEGESTOFFE

ARTICLE D'HYGIÈNE PERSONNELLE PERMETTANT DE DISTRIBUER SÉQUENTIELLEMENT DES COMPOSITIONS AVEC DES CONCENTRATIONS VARIABLES DE MATIÈRES BÉNÉFIQUES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **28.01.2009 US 361492**

(43) Date of publication of application:
**07.12.2011 Bulletin 2011/49**

(73) Proprietor: **The Procter & Gamble Company**
**Cincinnati, OH 45202 (US)**

(72) Inventors:
• **CETTI, Jonathan, Robert**
  **Mason, Ohio 45040 (US)**
• **PUTMAN, Christopher, Dean**
  **West Chester, Ohio 45069 (US)**

• **FARRIS, Richard, Duffy**
  **West Chester, Ohio 45069 (US)**
• **WEI, Karl, Shiqing**
  **Mason, Ohio 45040 (US)**
• **ABSHER, Sarah, Noelle**
  **Richmond**
  **Surrey TW10 6DQ (GB)**

(74) Representative: **Kremer, Véronique Marie Joséphine**
**Procter & Gamble Service GmbH**
**IP Department**
**Frankfurter Strasse 145**
**61476 Kronberg im Taunus (DE)**

(56) References cited:
**EP-A2- 0 243 321       FR-A1- 2 233 036**
**US-A1- 2006 079 417     US-A1- 2009 028 808**
**US-A1- 2009 028 809**

**Description**

FIELD OF THE INVENTION

The present invention relates to a personal care article that provides a personal care product with variable concentrations of hydrophobic benefit material throughout the package.

BACKGROUND OF THE INVENTION

Personal care compositions are well known and widely used for cleansing and moisturizing skin and hair, delivering actives, hiding imperfections, reducing the oiliness or shine of skin and hair, as well as, providing scent to the shower and/or the hair and skin. The efficacy of personal care compositions is directly related to the frequency of use and the level of benefit materials. In some cases, a high level of benefit materials in a personal care composition will maintain a benefit to a consumer for several days after a single application. In this case, a full bottle of the personal care composition with a high level of benefit materials is not needed because the continued application of this personal care composition would not provide additional benefits to the consumer over a single application or two applications. Numerous cosmetic applications require that the corresponding compositions are to be used at a variable dose of benefit materials over the course of time. Up until now, it order to carry out these treatments, the available resources have consisted either of successive applications of increasing or decreasing benefit material concentrations in separate packages or multiplying the applications of compositions with identical benefit material concentrations in order to obtain the correct does for the necessary treatment. If a treatment regime contains too many steps or too many packages, consumers often habituate or tire of the regime of personal care compositions over time. As a result, consumer may decrease or even or stop use of the regime of personal care products despite the benefits gained by the compliant use of this regime over time. With the space in the shower or bath being limited, a typical shower or bath does not have enough space, to store multiple packages of personal care compositions so that a consumer can easily switch the use of one personal care composition for another with a different concentration of benefit materials. Document EP-A-0243321 shows a package for sequentially dispensing doses of a personal care composition, the doses having progressively increasing active substance concentrations. Accordingly, there remains a need still remains for a personal care composition regime that simplified that provides excellent skin benefits. This need is solved by a method for dispensing a personal care composition comprising the features of claim 1

SUMMARY OF THE INVENTION

A method for dispensing a personal care composition is disclosed. The personal care composition is formed from two compositions, one of which includes a surfactant and one of which includes a lipid. The method includes dispensing a first dose from the package, wherein the first dose has a concentration of the lipid by weight of the first dose, and dispensing a second dose after the first dose, wherein the second dose has a concentration of the lipid by weight of the second dose that is greater than the concentration of the lipid by weight of the first dose. A third dose is dispensed after the second dose, wherein the third dose has a concentration of the lipid by weight of the third dose that is less than the concentration of the lipid by weight of the second dose.

BRIEF DESCRIPTION OF THE DRAWINGS

FIG. 1A and FIG. 1B illustrate a personal care article with three zones having horizontal interfaces between the compositions in each zone.

FIG. 2A is a diagram of the distinguishable layers of a personal care product after centrifugation which can be measured in length to calculate the concentration of hydrophobic benefit material in the personal care product using the Microcentrifugation Method described below.

FIG. 2B and FIG. 2C are photographs that exemplify the measurement of the length of the benefit layer used to calculate the concentration of the hydrophobic benefit material within in centrifuged samples tested using the Microcentrifugation Method described below.

FIG. 3 is a calibration curve calculated using a formula in the Microcentrifugation Method described below.

FIG. 4A and FIG. 4B illustrate a graphic user interface analysis of a personal care product phase distribution along the radial dimensions of the package according to the MRI method described below.

FIG. 5A and FIG. 5B illustrate a graphic user interface analysis of a personal care product phase distribution along the height of the package according to the MRI method described below.

FIG. 6A, FIG. 6B, and FIG. 6C are MRI images of hydrophobic benefit material distribution profiles prior to and after simulated shipping conditions, as per the Dynamic Stability Shipping Method described below.

FIG. 7A, FIG. 7B and FIG. 7C are MRI images of hydrophobic benefit material distribution profiles of personal care products described in the examples below.

FIG. 8 is a chart showing the benefit phase distribution profile of the hydrophobic benefit material in the personal care products described in the examples below.

FIG. 9 is a front elevational view of a package that is separable into a plurality of sections.

FIG. 10 is a front elevation view of the package of FIG. 9, wherein the plurality of sections have been separated from each another.

FIG 11 is a schematic illustration of a manufacturing line comprising a filling station for filling a plurality of packages simultaneously.

DETAILED DESCRIPTION OF THE INVENTION

The term "ambient conditions" as used herein, refers to surrounding conditions at one (1) atmosphere of pressure, 50% relative humidity, and 25°C.

As used herein, "comprising" means that other steps and other ingredients which do not affect the end result can be added. This term encompasses the terms "consisting of" and "consisting essentially of." The compositions and methods/processes of the present invention can comprise, consist of, or consist essentially of the essential elements and limitations of the invention described herein, as well as any of the additional or optional ingredients, components, steps, or limitations described herein useful in personal cleansing compositions intended for topical application to the hair or skin.

The term "headspace," as used herein means the void volume that is located proximal to the dispensing orifice and the interface of the first zone of the single chamber package. In the alternative, the headspace can be comprised within the first zone. The headspace of the personal care articles of the present invention can be determined by the following method or any other conventional method. First, an empty package is placed on a balance and weighed. The total package volume is determined by completely filling the package with deionized water and determining the deionized water weight and recording it as ($V_{total}$). The package is then filled with a personal care composition leaving a headspace. Next, the package is placed on a balance and re-zero. The headspace volume is filled with deionized water by a syringe. The weight of deionized water filled in the headspace is recorded as ($V_{headspace}$). The headspace is calculated as: $Vh_{eadspace}/V_{total}*100\%$.

The term "liquid" as used herein means that the composition is generally flowable to some degree. "Liquids", therefore, may include liquid, semi-liquid, cream, lotion or gel compositions intended for topical application to skin. The compositions may exhibit a viscosity of equal to or greater than about 1,500 (centipoise, hereinafter "cps"), equal to or greater than about 5,000 cps, equal to or greater than about 10,000 cps or equal to or greater than about 20,000 cps and no more than about 1,000,000 cps, no more than about 500,000 cps, no more than about 300,000 cps, or no more than about 200,000 cps as measured by the T-Bar Viscosity Method described hereinafter.

The term "package" includes any suitable container for personal care compositions exhibiting a viscosity from about 1,500 centipoise (cP) to about 1,000,000 cP, including but not limited to a bottle, tottle, tube, jar, non-aerosol pump and mixtures thereof. As used herein "tottle" refers to a bottle which rests on the neck or mouth which its contents are filled in and dispensed from, but it is also the end upon which the bottle is intended to rest or sit upon for storage by the consumer and/or for display on the store shelf, as described in the commonly owned U.S. Patent Application Serial No, 11/067443 filed on Feb. 25, 2005 to McCall et al, entitled "Multi-phase Personal Care Compositions, Process for Making and Providing, and Article of Commerce."

The term "personal care composition" as used herein, refers to compositions intended for topical application to the skin or hair. The compositions of the present invention are rinse-off formulations, in which the product is applied topically to the skin or hair and then is subsequently rinsed within minutes from the skin or hair with water, or otherwise wiped off using a substrate with deposition of a portion of the composition. The compositions also may be used as shaving aids. The personal care composition of the present invention is typically extrudable or dispensible from a single chamber package. The personal care compositions of the present invention can be in the form of liquid, semi-liquid, cream, lotion or gel compositions intended for topical application to skin. Examples of personal care compositions of the present invention can include but are not limited to shampoo, conditioning shampoo, hair conditioner, body wash, moisturizing body wash, shower gels, skin cleansers, cleansing milks, hair and body wash, in shower body moisturizer, pet shampoo, shaving preparations and cleansing compositions used in conjunction with or applied to a disposable cleansing cloth. The product forms contemplated for purposes of defining the compositions and methods of the present invention are rinse-off invention are rinse-off formulations by which it is meant that the product is applied topically to the skin or hair and then subsequently (i.e., within minutes) rinsed away with water, or otherwise wiped off using a substrate or other suitable removal means.

The term "statically stable" as used herein, unless otherwise specified, refers to a personal care product that comprise at least two compositions that maintain at least two "separate" zones with at least two separate benefit concentrations zones contained within a single chamber package at ambient conditions for a period of at least about 180 days. Alternatively, static stability can be determined by accelerated protocol at elevated temperature. One accelerated protocol is based on passing static stability after 10 days at 50°C. By "separate" is meant that there is substantially no mixing of compositions contained in the zones, detected by the benefit analysis method, described hereinafter, prior to dispensing of the composition.

The term "structured," as used herein means having a rheology that confers stability on the personal care composition. The degree of structure is determined by characteristics determined by one or more of the following methods the Yield Stress Method, or the Zero Shear Viscosity Method or by the Ultracentrifugation Method, all in the Test Methods below. Accordingly, a surfactant phase of the composition of the present invention is considered "structured," if the surfactant phase has one or more of the following properties described below according to the Yield Stress Method, or the Zero Shear Viscosity Method or by the Ultracentrifugation Method. A surfactant phase is considered to be structured, if the phase has one or more of the following characteristics:

A. a Yield Stress of greater than about 0.1 Pascal (Pa), more preferably greater than about 0.5 Pa, even more preferably greater than about 1.0 Pa, still more preferably greater than about 2.0 Pa, still even more preferably greater than about 3 Pa, and even still even more preferably greater than about 5 Pa as measured by the Yield Stress and Zero Shear Viscosity Method described hereafter:

B. a Zero Shear Viscosity of at least about 500 Pascal-seconds (Pa-s), preferably at least about 1,000 Pa-s, more preferably at least about 1,500 Pa-s, even more preferably at least about 2,000 Pa-s; or

C. a Structured Domain Volume Ratio as measured by the Ultracentrifugation Method described hereafter, of greater than about 40%, preferably at least about 45%, more preferably at least about 50%, more preferably at least about 55%, more preferably at least about 60%, more preferably at least about 65%, more preferably at least about 70%, more preferably at least about 75%, more preferably at least about 80%, even more preferably at least about 85%.

The term "surfactant component" as used herein means the total of all anionic, nonionic, amphoteric, zwitterionic and cationic surfactants in a phase. When calculations are based on the surfactant component, water and electrolyte are excluded from the calculations involving the surfactant component, since surfactants as manufactured typically are diluted and neutralized.

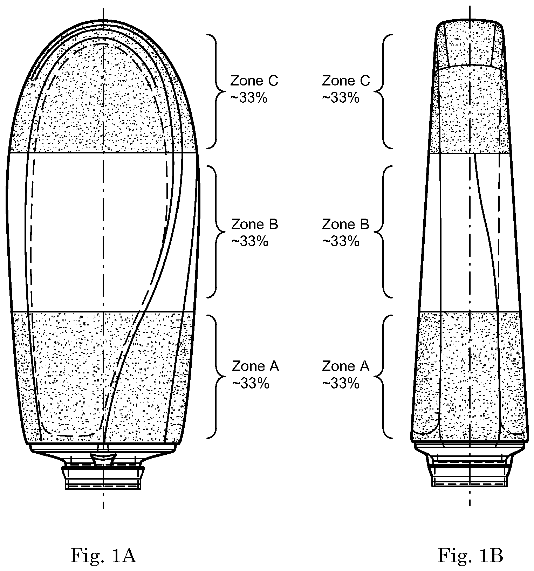

As used herein the term "zone" is a domain or region within a single chamber package which corresponds to a composition of the personal care product. The interface between the zones can be distinct or gradual or separated by another zone. The amount contained within a zone can be defined by a percentage of the package volume and a zone comprises at least 10% of the package volume of a given package, excluding the volume of the package corresponding to the necessary headspace or void volume and the closure, as shown in FIG. 1A and FIG. 1B of the present invention. In one aspect, the first personal care composition, the second personal care composition and third personal care compositions within a the first zone, second zone or third zone is homogeneous. In this case, the concentration of hydrophobic benefit material is constant within the zone. In another aspect, the personal care composition within the first, second or third zone is inhomogeneous, such that the concentration of hydrophobic benefit material varies within the zone. The level of hydrophobic benefit material can show an increasing or decreasing trend.

All percentages, parts and ratios are based upon the total weight of the compositions of the present invention, unless otherwise specified. All such weights as they pertain to listed ingredients are based on the active level and, therefore; do not include solvents or by-products that may be included in commercially available materials, unless otherwise specified. The term "weight percent" may be denoted as "wt. %" herein. Except where specific examples of actual measured values are presented, numerical values referred to herein should be considered to be qualified by the word "about."

All molecular weights as used herein are weight average molecular weights expressed as grams/mole, unless otherwise specified.

The present invention relates to personal care article for dispensing a personal care product that comprises preferably a single chamber package and a personal care product. The single chamber package comprises a dispensing orifice, a first zone proximal to the dispensing orifice, a second zone medial to the dispensing orifice, and a third zone distal to the dispensing orifice. The personal care product comprises a first personal care composition, a second personal care composition and a third personal care composition. The first personal care composition is substantially within the first zone and comprises a first concentration of a hydrophobic benefit material. The second personal care composition is substantially within the second zone and comprises a second concentration of a hydrophobic benefit material. The third personal care composition is substantially within the third zone and comprises a third concentration of a hydrophobic benefit material. The second concentration is greater than the first concentration and the third concentration of the hydrophobic benefit material. The first personal care composition is capable of being substantially dispensed prior to the second personal care composition and the third personal care composition. The second personal care composition is capable of being substantially dispensed prior to the third personal care composition.

The personal care product of the present invention, in most embodiments, is statically stable. In most embodiments, the personal care product of the present invention is dynamically stable according to the Dynamic Stability Shipping Method disclosed in the Test Methods below.

In some embodiments, the first personal care composition is in physical contact with the second personal care composition within the single chamber package. The second personal care composition, in another embodiment, is in physical contact with the third personal care composition within the single chamber package.

In one embodiment, the first zone, second zone and/or third zone of the present invention comprises from about 10% to about 70%, by volume, of the package. The first zone, second zone and/or third zone of the present invention comprise from about 10% to about 60%, from about 10% to about 50%, from about 10% to about 40%, from about 10% to about 30%, from about 10% to about 20%, by volume, of the package. In other embodiments, the first zone, second zone and/or third zone of the present invention comprises from about 20% to about 70%, from about 20% to about 60%, from about 20% to about 50%, from about 20% to about 40%, from about 20% to about 30%, by volume, of the package. In other embodiments, the first second and/or third zone of the present invention comprises from about 30% to about 70%, from about 30% to about 60%, from about 30% to about 50%, from about 30% to about 40%, by volume, of the package.

The personal care article of the present invention preferably comprises a single chamber package which can contain any number or zones and compositions, such as for example, four zones and four compositions, five zones and five compositions, six zones and six compositions, twelve zones and twelve compositions, and so on. Each of these compositions is capable of substantially dispensing prior to the composition before it in a substantially sequential manner. For example, the fourth personal care composition substantially within the fourth zone is capable of dispensing prior to the fifth personal care composition within the fifth zone, etc.

The personal care article of the present invention is filled to comprise a headspace. In some embodiments, the personal care article of the present invention comprises a headspace that is less than 10%, is less than 6%, less than 5% and less than 4%. In other embodiments, the personal care article of the present invention comprises a headspace that is less than 3%, less than 2% and less than 1%.

In another aspect, each personal care composition comprises a dye, colorant or the like, such that each personal care composition is a distinct color or hue. For example, the first personal care composition is a yellow color, the second personal care composition is an orange color and the third personal care composition is a purple color.

The amount or concentration of hydrophobic benefit materials in the first personal care composition, second personal care composition and third personal care composition are usually formulated, by weight of the composition, at less than about 75%, less than about 65%, less than about 60%, less than about 60%, less than about 55%, less than about 50%, less than about 45%, less than about 40%, less than about 35%, less than about 30%, less than about 25%, less than about 20%, less than about 10%, less than about 5%, less than about 4%, less than about 3%, less than about 2%, less than about 1%. The first personal care composition, second personal care composition and third personal care composition comprises from about 1.0% to about 60%, from about 5% to about 60%, from about 10% to about 50%, from about 20% to about 45%, by weight of the personal care composition, of a hydrophobic benefit material.

In some embodiments, the first concentration can comprise from about 10% to less than about 50% or, from about 10% to about 40%, by weight of the first personal care composition. The first concentration of hydrophobic material, in other embodiments, comprise from about 15% to less than 45% or 15% to less than 35% by weight of the first personal care composition, of hydrophobic benefit material. The first concentration, in some embodiments, comprise from about 20% to about 40% and from about 25% to about 40%, by weight of the first personal care composition.

In some embodiments, the second concentration comprises from greater than 30% to about 70%, greater than about 35% to about 65%, by weight of the second personal care composition, of hydrophobic benefit material. In another embodiment, the second concentration comprises from about 40 to about 60% and about 55% by weight of the second personal care composition.

In some embodiments, the third concentration can comprise from about 10% to less than about 50% or, from about 10% to about 40%, by weight of the third personal care composition. The third concentration of hydrophobic material, in other embodiments, comprise from about 15% to less than 45% or 15% to less than 35% by weight of the third personal care composition, of hydrophobic benefit material. The third concentration, in some embodiments, comprise from about 20% to about 40% and from about 25% to about 40%, by weight of the third personal care composition.

In one embodiment, the first personal care composition, second personal care composition and third personal care composition of the present invention are multi-phase compositions and comprise one of more phases or one or more of the components described in the phases below:

The personal care compositions of the present invention comprise a benefit phase or benefit phase components. The benefit phase in the present invention, in most embodiments, is anhydrous and is substantially free of water. In some embodiments, the benefit phase is substantially free or free of surfactant.

Hydrophobic benefit materials suitable for use in the present invention preferably have a Vaughan Solubility Parameter of from about 5 $(cal/cm^3)^{1/2}$ to about 15 $(cal/cm^3)^{1/2}$, as defined by Vaughan in Cosmetics and Toiletries, Vol. 103. The Vaughan Solubility Parameter (VSP) as used herein is a parameter used to define the solubility of hydrophobic materials. Vaughan Solubility parameters are well known in the various chemical and formulation arts and typically have a range of from 5 to 25. Non-limiting examples of hydrophobic benefit materials having VSP values ranging from about 5 to about 15 include the following: Cyclomethicone 5.92, Squalene 6.03, Petrolatum 7.33, Isopropyl Palmitate 7.78, Isopropyl Myristate 8.02, Castor Oil 8.90, Cholesterol 9.55, as reported in Solubility, Effects in Product, Package, Penetration and Preservation, C. D. Vaughan, Cosmetics and Toiletries, Vol. 103, October 1988.

The hydrophobic benefit materials for use in the benefit phase of the composition have a preferred rheology profile as defined by Consistency value (k) and Shear Index (n). The term "Consistency value" or "k" as used herein is a measure of lipid viscosity and is used in combination with Shear Index, to define viscosity for materials whose viscosity is a function of shear. The measurements are made at 35°C and the units are poise (equal to 100 cps). The term "Shear Index" or "n" as used herein is a measure of lipid viscosity and is used in combination with Consistency value, to define viscosity for materials whose viscosity is a function of shear. The measurements are made at 35°C and the units are dimensionless. Consistency value (k) and Shear Index (n) are more fully described in the Test Methods below. Preferred Consistency value ranges are 1-10,000 poise $(1/sec)^{n-1}$, preferably 10-2000 poise $(1/sec)^{n-1}$ and more preferably 50-1000 poise $(1/sec)^{n-1}$. Shear Index ranges are 0.1-0.8, preferably 0.1-0.5 and more preferably 0.20-0.4. These preferred rheological properties are especially useful in providing the personal cleansing compositions with improved deposition of benefit agents on skin.

In one embodiment, the benefit phase is comprised of the hydrophobic benefit materials selected from the group consisting of petrolatum, lanolin, derivatives of lanolin (e.g. lanolin oil, isopropyl lanolate, acetylated lanolin, acetylated lanolin alcohols, lanolin alcohol linoleate, lanolin alcohol riconoleate) hydrocarbon oils (e.g. mineral oil) natural and synthetic waxes (e.g. micro-crystalline waxes, paraffins, ozokerite, lanolin wax, lanolin alcohols, lanolin fatty acids, polyethylene, polybutene, polydecene, pentahydrosqualene) volatile or non-volatile organosiloxanes and their derivatives (e.g. dimethicones, cyclomethicones, alkyl siloxanes, polymethylsiloxanes, methylphenylpolysiloxanes), natural and synthetic triglycerides (e.g. castor oil, soy bean oil, sunflower seed oil, maleated soy bean oil, safflower oil, cotton seed oil, corn oil, walnut oil, peanut oil, olive oil, cod liver oil, almond oil, avocado oil, palm oil, sesame oil) and combinations thereof. In one aspect, at least about 50% by weight of the hydrophobic benefit materials are selected from the groups of petrolatum, mineral oil, paraffins, polyethylene, polybutene, polydecene, dimethicones, alkyl siloxanes, cyclomethicones, lanolin, lanolin oil, lanolin wax. In one embodiment, the remainder of the hydrophobic benefit material is selected from: isopropyl palmitate, cetyl riconoleate, octyl isononanoate, octyl palmitate, isocetyl stearate, hydroxylated milk glyceride and combinations thereof. The benefit phase of the personal care composition, in some embodiments, comprises a combination of petrolatum and mineral oil.

In some embodiments, the personal care composition of the present invention comprises a surfactant phase. The personal care composition typically comprises from about 1 % to about 100 %, by weight of the composition; from about 5% to about 85%; by weight of the composition, from about 10% to 80%, by weight of the composition; from about 20 to 70%, by weight of the composition; from about 25% to 60%, by weight of the composition, from about 30% to about 50%, by weight of the composition, of a surfactant phase.

In some embodiments, the surfactant phase comprises a structured domain that is comprised of a mixture of surfactants. The presence of structured domain enables the incorporation of high levels of hydrophobic benefit materials in a separate phase which is not emulsified within composition. In one aspect, the structured domain in the composition is characterized as, or is, an opaque structured domain. In one aspect, the opaque structured domain is characterized as, or is, a lamellar phase. The lamellar phase produces a lamellar gel network. The lamellar phase provides resistance to shear, adequate yield to suspend particles and droplets and at the same time provides long term stability, since it is thermodynamically stable. The lamellar phase tends to have a higher viscosity thus minimizing the need for viscosity modifiers.

In one aspect, the surfactant phase comprises a domain that is comprised of a mixture of surfactants and is a micellar phase. A micellar phase is optically isotropic. Micelles are approximately spherical in shape. Other shapes such as ellipsoids, cylinders, and bilayers are also possible. In one aspect, the micellar phase is structured to enhance viscosity and to suspend particles. This can be accomplished using viscosity modifiers such as those defined below as water structurants.

In some embodiments, the surfactant phase comprises a surfactant component which comprises of a mixture of surfactants including lathering surfactants or a mixture of lathering surfactants. The surfactant phase comprises surfactants suitable for application to the mammalian skin or hair and is compatible with water and the other ingredients of the composition of the present invention. These surfactants include anionic, nonionic, cationic, zwitterionic, amphoteric, soap, or combinations thereof. Preferably, anionic surfactant comprises at least 40% of the surfactant component. The personal care composition, in some embodiments, comprises the surfactant component at concentrations ranging from about 2% to about 40%, from about 4% to about 25%, about 1% to about 21%, about 3 to 15%, by weight of the composition, of the surfactant component.

Suitable surfactants are described in McCutcheon's, Detergents and Emulsifiers, North American edition (1986), published by allured Publishing Corporation; and McCutcheon's, Functional Materials, North American Edition (1992); and in U.S. Pat. No. 3,929,678 issued to Laughlin, et al on December 30, 1975.

Preferred linear anionic surfactants for use in the surfactant phase of the personal care composition include ammonium lauryl sulfate, ammonium laureth sulfate, sodium lauryl sulfate, sodium laureth sulfate, potassium laureth sulfate, sodium lauryl sarcosinate, sodium lauroyl sarcosinate, lauryl sarcosine, cocoyl sarcosine, ammonium cocoyl sulfate, potassium lauryl sulfate, and combinations thereof.

Branched anionic surfactants and monomethyl branched anionic surfactants suitable for the present invention are described in a commonly owned U.S. Publication No. 60/680,149 entitled "Structured Multi-phased Personal Cleansing Compositions Comprising Branched Anionic Surfactants" filed on May 12, 2005 by Smith, et al. Branched anionic surfactants include but are not limited to the following surfactants: sodium trideceth sulfate, sodium tridecyl sulfate, sodium $C_{12}$-$_{13}$ alkyl sulfate, and $C_{12}$-$_{13}$ pareth sulfate and sodium $C_{12}$-$_{13}$ pareth-*n* sulfate.

In one aspect of the personal care compositions of the present invention comprise an amphoteric surfactant, a zwitterionic surfactant and mixtures thereof. In one embodiment, the personal care composition comprises at least one amphoteric surfactant. Amphoteric surfactant suitable for use in the present invention include those that are broadly described as derivatives of aliphatic secondary and tertiary amines in which the aliphatic radical can be straight or branched chain and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Pat. No. 2,658,072, N-higher alkyl aspartic acids such as those produced according to the teaching of U.S. Pat. No. 2,438,091, and the products described in U.S. Pat. No. 2,528,378. In one aspect, the personal care composition comprises an amphoteric surfactant that is selected from the group consisting of sodium lauroamphoacetate, sodium cocoamphoactetate, disodium lauroamphoacetate disodium cocodiamphoacetate, and mixtures thereof. Moreover, Amphoacetates and diamphoacetates are also used in some embodiments of the present invention.

Zwitterionic surfactants suitable for use include those that are broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate. Zwitterionic surfactants suitable for use in the personal care composition include alkyl betaines, including cocoamidopropyl betaine.

The personal care composition of the present invention is preferably free of alkyl amines and alkanolamide to ensure mildness of the composition to the skin.

An electrolyte can be added per se to the personal care composition or it can be formed in situ via the counterions included in one of the raw materials. The electrolyte preferably includes an anion comprising phosphate, chloride, sulfate or citrate and a cation comprising sodium, ammonium, potassium, magnesium or mixtures thereof. Some preferred electrolytes are sodium chloride, ammonium chloride, sodium or ammonium sulfate. The electrolyte is preferably added to the surfactant phase of the composition in the amount of from about 0.1% to about 6%; from about 1% to about 5%, more preferably from about 2% to about 4%, more preferably from about 3% to about 4%, by weight of the personal care composition.

In one embodiment, the first personal care composition comprise a first concentration of surfactant, the second personal care composition comprises a second concentration of surfactant and the third personal care composition comprises a third concentration of surfactant. The first concentration of surfactant is different from the second concentration of surfactant and the third concentration of surfactant, in some embodiments. In one aspect, the first personal care composition has a first concentration of surfactant that is a greater that the second concentration of surfactant in the second personal care compositions and is the same as or greater than the third concentration of surfactant in the third personal care compositions. In one aspect, the first personal care composition has a lower concentration of surfactant than the second and the third personal care compositions.

In some embodiments, the personal care compositions of the present invention comprise a structured aqueous phase. The structured aqueous phase, in one embodiment, comprises a water structurant and water. The structured aqueous phase has a pH in the range from about 5 to about 9.5, or in one aspect have a pH of about 7. In one embodiment, the structured aqueous phase is hydrophilic. In one aspect, the structured aqueous phase is a hydrophilic, non-lathering gelled water phase.

In some embodiments, the structured aqueous phase comprises less than about 5%, less than about 3%, less than about 1%, by weight of the structured aqueous phase, of a surfactant component. In one aspect, the structured aqueous phase is free of lathering surfactants in the composition. In one embodiment, the structured aqueous phase of the present invention comprises from about 30% to about 99%, more than about 50%, more than about 60%, more than about 70%, more than about 80%, by weight of the structured aqueous phase, of water.

In one embodiment, the structured aqueous phase comprises a water structurant. The water structurant is selected from the group consisting of inorganic water structurants (e.g. silicas, polyacrylates, polyacrylamides, modified starches, crosslinked polymeric gellants, copolymers) charged polymeric water structurants (e.g. Acrylates/Vinyl Isodecanoate Crosspolymer available, STABYLEN 30® available from 3V SIGMA S.P.A of Bergamo Italy), Acrylates/C10-30 Alkyl Acrylate Crosspolymer (e.g. PEMULEN ™ TR1 and TR2 polymers available from NOVEON®), Carbomers, Ammonium Acryloyldimethyltaurate/VP Copolymer (e.g. Aristoflex® AVC available from Clariant), Ammonium Acryloyldimethyltaurate/Beheneth-25 Methacrylate Crosspolymer (e.g. ARISTOFLEX® HMB available from Clariant), Acrylates/Ceteth-20 Itaconate Copolymer (e.g. STRUCTURE® 3001 available from National Starch), Polyacrylamide (e.g. SEPIGEL™ 305 available from SEPPIC), water soluble polymeric structurants (e.g. cellulose gums and gel, and starches), associative water structurants (e.g. xanthum gum, gellum gum, pectins, alginates such as propylene glycol alginate), and mixtures thereof. In some embodiments, the structured aqueous phase comprises from about 0.1% to about 30%, from about 0.5% to about 20%, from about 0.5% to about 10%, and from about 0.5% to about 5%, by weight of the structured aqueous phase, of a water structurant. In some embodiments, a water structurant for the structured aqueous phase has a net cationic charge, net anionic charge, or neutral charge.

While not essential for the purposes of the present invention, the non-limiting list of optional materials, illustrated hereinafter are suitable for use in personal care compositions, and may be incorporated in certain embodiments, for example to assist or enhance cleansing performance, for treatment of the skin, or to modify the aesthetics of the personal care composition. Optional materials useful in the products herein are described by their cosmetic and/or therapeutic benefit or their postulated mode of action or function. These descriptions are non-limiting and made for the sake of convenience because it is understood that these materials can provide more than one benefit, function or operate via more than one mode of action. The precise nature of these optional materials, and levels of incorporation thereof, will depend on the physical form of the composition and the nature of the cleansing operation for which it is to be used. The amount of optional materials in compositions are usually formulated, by weight of the composition, at less than about less than about 6%, less than about 5%, less than about 4%, less than about 3%, less than about 2%, less than about 1%, less than about 0.5%, less than about 0.25%, less than about 0.1%, less than about 0.01%, less than about 0.005%.

In some embodiments of the present invention, comprise optional ingredients, which are selected from the group consisting of thickening agents, low density microspheres (e.g. EXPANCEL® microspheres available from 091 WE40 d24, Akzo Nobel and others described in commonly owned and assigned U.S. Patent Publication No. 2004/0092415A1 published on May 13, 2004), preservatives, antimicrobials, fragrances, chelators (e.g. such as those described in U.S. Pat. No. 5,487,884 issued to Bisset et al.), sequestrants, vitamins (e.g. Retinol), vitamin derivatives (e.g. tocophenyl actetate, niacinamide, panthenol), sunscreens, desquamation actives (e.g. such as those described in U.S. Pat. No. 5,681,852 and 5,652,228 issued to Bisset), anti-wrinkle/ anti-atrophy actives (e.g. N-acetyl derivatives, thiols, hydroxyl acids, phenol), antioxidants (e.g. ascorbic acid derivatives, tocophenol), skin soothing agents/skin healing agents (e.g. panthenoic acid derivatives, aloe vera, allantoin), skin lightening agents (e.g. kojic acid, arbutin, ascorbic acid derivatives), skin tanning agents (e.g. dihydroxyacteone), polymeric phase structurant (e.g. naturally derived polymers, synthetic polymers, crosslinked polymers, block copolymers, copolymers, hydrophilic polymers, nonionic polymers, anionic polymers, hydrophobic polymers, hydrophobically modified polymers, associative polymers, and oligomers); a liquid crystalline phase inducing structurant (e.g. trihydroxystearin available from Rheox, Inc. under the trade name THIXCIN® R), organic cationic deposition polymer (e.g. Polyquaternium 10 available from Amerchol Corp., guar hydroxypropyltrimonium chloride available as JAGUAR® C-17 from Rhodia Inc., and N-HANCE® polymer series commercially available from AQUALON), pH regulators (e.g. triethanolamine), anti-acne medicaments, essential oils, sensates, pigments, colorants, pearlescent agents, interference pigments (e.g. such as those disclosed in U.S. Pat. No. 6,395,691 issued to Liang Sheng Tsaur, U.S. Pat. No. 6,645,511 issued to Aronson et al., U.S. Pat. No. 6,759,376 issued to Zhang et al., U.S. Pat. No. 6,780,826 issued to Zhang et al.) particles (e.g. talc, kolin, mica, smectite clay, cellulose powder, polysiloxane, silicas, carbonates, titanium dioxide, polyethylene beads) hydrophobically modified non-platelet particles (e.g. hydrophobically modified titanium dioxide and other materials described in a commonly owned, patent application published on Aug. 17, 2006 under Publication No. 2006/0182699A by Taylor, et al.) and mixtures thereof. Other optional ingredients are most typically those materials approved for use in cosmetics and that are described in the CTFA Cosmetic Ingredient Handbook, Second Edition, The Cosmetic, Toiletries, and Fragrance Association, Inc. 1988, 1992.

TEST METHODS

Microcentrifugation Method:

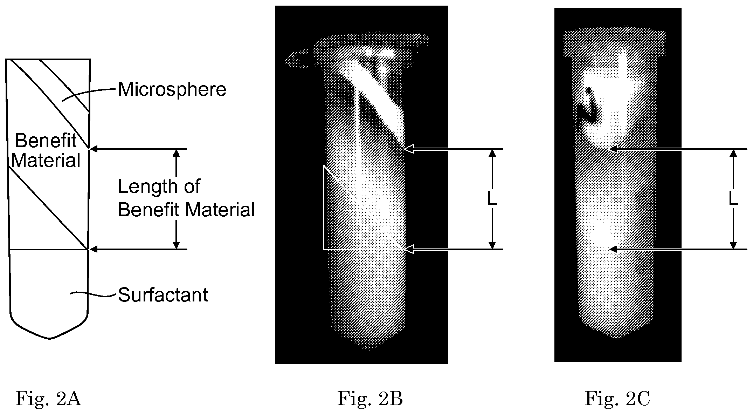

The Microcentrifugation Method determines the variation of the percent of hydrophobic benefit material per dose in a package that comprises a personal care product. As an overview, the personal care products being tested are dispensed in 10.0 mL sample sizes and these samples are centrifuged. Centrifugation separates the sample size of personal care products into distinguishable layers. The first personal care composition, second personal care composition and third personal care composition have multiple distinguishable layers, for example a microsphere layer, a surfactant layer, and a benefit layer that comprises hydrophobic benefit material, as shown in FIG. 2B and FIG. 2C. After centrifugation, the volume percentage of the benefit phase for each sample is determined and plotted per dose of personal care product to obtain the hydrophobic benefit material distribution profile of the personal care product throughout the product package.

| Table 1: Description of Apparatus used in the Microcentrifugation Method | |
|---|---|
| Apparatus: | Description: |
| Micro-centrifuge | VWR Galaxy 16DH |
| 2mL Micro-centrifuge clear tubes | VWR cat. No. 20170-170 |
| Disposable syringes | 1mL, VWR cat. No. BD309602 |
| Top Load balance | Capable of weighing to 2 decimal cases. |
| Clear plastic cups | 207mL Solo Plastic cup |
| Centrifuge tube stand | capacity to hold at least 24 tubes |
| Electronic Digital Caliper | capable of measuring 2 decimal cases in mm |

To prepare the samples for a 295mL package of a personal care product, label 24 clear plastic cups 1-24. Place cup 1 on top of balance and tare. Open package containing the personal care product, dispense 8.80 g $\pm$ 0.50g of product in cup 1, and record the weight of each sample. Repeat these instructions for all 24 cups, or for as many doses you can get from one package. If composition gets stuck in the package, tap the package in descending motion for four times.

Next, label 24 centrifuge tubes 1-24 doses. Then, mix the sample in cup 1 well by stirring the sample with a stirrer by hand and then draw the sample into a syringe. Insert the syringe all the way to the bottom of the centrifuge tube. Slowly push the plunger as you withdraw the syringe form the centrifuge tube, making sure no air bubbles or gaps are formed. Check for air bubbles, if any air bubble is found tap the centrifuge tube until sample fills the gaps left by the air bubbles. Load the syringe with more sample of the product and bring the extremity of the syringe to the top of the sample of the product that is inside the centrifuge tube. Slowly push the plunger while withdrawing the syringe from the centrifuge tube. Check for air bubbles, and eliminate them by tapping down the centrifuge tube. Fill the centrifuge tube to its maximum capacity with the sample of the product (i.e. all the way to the rim), cap the centrifuge tube and place in the centrifuge tube rack. Repeat these steps until all 24 centrifuge tubes are filled.

Load the centrifuge as described in the manufacturer's instrument operation section of the instruction manual. Centrifuge each of the samples for 15 minutes at 13,000 rpm. Once centrifugation is done, remove each centrifuge tube from centrifuge. Next, use a caliper to measure the length of benefit phase to 1/100 of mm. Record the length of benefit phase for each sample.

FIG. 2A is a diagram of the layers of a personal care composition after centrifugation. FIG. 2B and FIG. 2C are photographs that exemplify the measurement of the benefit phase comprising hydrophobic benefit material within in the centrifuged samples.

The volume of each dispensed sample is calculated by convert the weight of each sample to volume using product density (0.88 g/mL).

$$\text{Volume}_{(sample)} = \frac{\text{Weight}}{\text{Density}}$$

The total volume dispensed is calculated by adding the volume of a sample to the sum of the volumes of all previous samples. The percent hydrophobic benefit material is calculated using equation of calibration curve, below. In this equation, y = length of benefit layer and x = the percentage hydrophobic benefit material in the sample.

$$X = \frac{y + 3.0416}{0.3867}$$

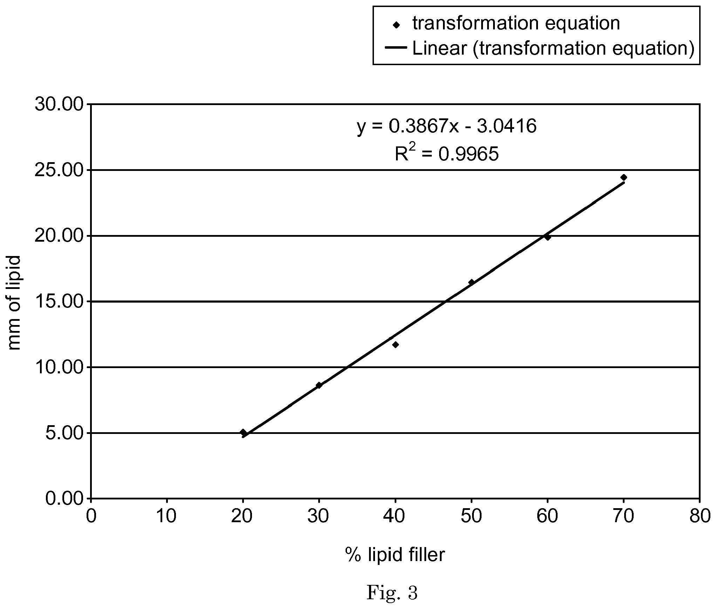

FIG. 3 depicts a calibration curve that was generated from 20 to 70% concentration of hydrophobic benefit material. This curve was used to transform mm of hydrophobic benefit material to percent of hydrophobic benefit material in the composition.

Finally, plot the percentage of hydrophobic benefit material versus the total volume dispensed to obtain the hydrophobic benefit material distribution profile of the personal care product throughout the package.

Magnetic Resonance Imaging (MRI) Method:

The MRI Method is used to obtain images and quantitatively describe the benefit distribution in 3-Dimension. The Instrument used is a 4.7T Magnex Scientific magnet with a 60 cm horizontal bore. The data is collected using a Bruker 25 cm imaging coil and Bruker Paravision 3.0.2. The data is collected using a spin-echo pulse sequence, repetition time of 1000 ms and echo time of 15 ms. Images were acquired of 32 of 2mm thick slices were acquired along the flat surface of the package or bottle. The fields of view were 22 cm x 10 cm with data size of 256 x 128, which results in in-plane pixel resolutions of 86 um x 78 um.

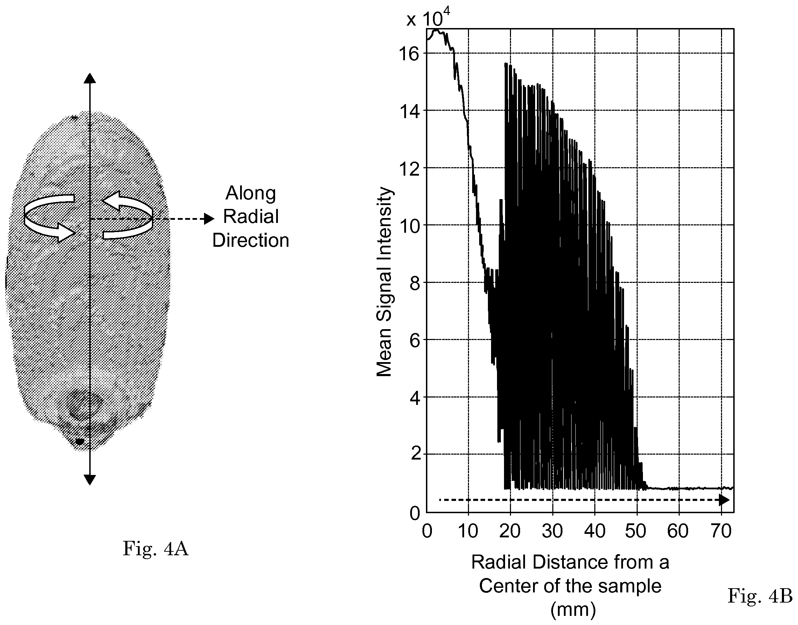

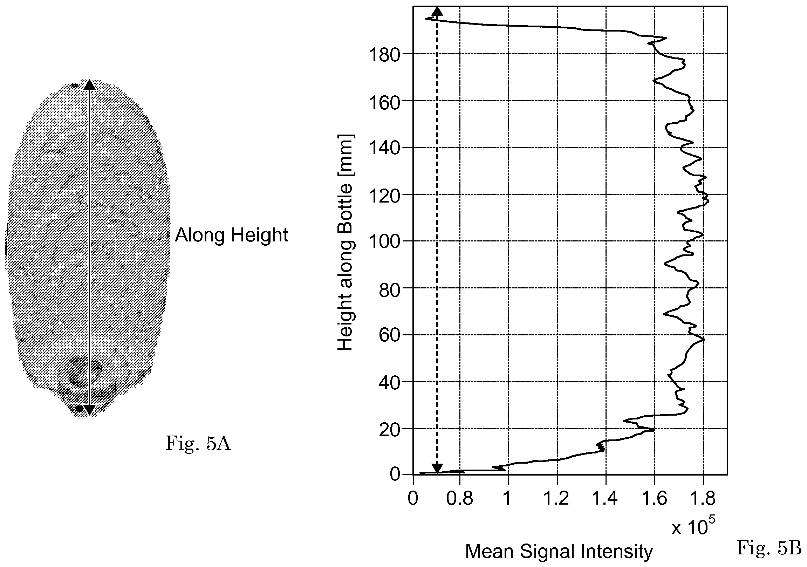

The customized imaging analysis software used to analyze the MRI images is a Matlab based graphical user interface program, hereinafter referred to as "GUI program". This GUI program was developed in order to quantitatively describe benefit layer distribution in 3-dimensions. The GUI program sets thresholds based on MRI intensity to segment background and/or void region, benefit region and surfactant region. The distribution of hydrophobic benefit material along the height and radial are summed and plotted as FIG. 4 and FIG. 5. FIG. 4 illustrates GUI based analysis of personal care composition phase distribution along the radial dimensions of the package. FIG. 5 illustrates GUI based analysis of personal care composition phase distribution along the height of the package.

Dynamic Shipping Stability Method:

The dynamic shipping stability method is a simulated shipping test that is conducted to illustrate the impact of the amount headspace on the distribution profile of hydrophobic benefit material in a personal care article of the present invention. The method is conducted on a vibration table, such as a MTS Vibration Table, available from Lansmont TTV of Monterrey, California.

The method tests shipping cases of personal care articles. There are 6 personal care articles or packages that are comprised within a shipping case. The personal care articles are filled with inventive example B using inventive filler profile B with various headspaces at 16%, 10% and 3%, of the volume of the personal care article, respectively. The shipping cases are submitted to simulated shipping conditions. The temperature of the shipping cases of personal care articles can be varied to simulate shipping conditions from cold to warm climate regions.

Prior to submitting the shipping cases to simulated shipping conditions, MRI images of each personal care articled are obtained by the MRI method at 25°C. Next, the shipping cases are subjected to simulated shipping conditions

There are four steps to induce the simulated shipping conditions:

Step 1: The shipping cases are dropped once at each of the six orientations for a total of six times. The "six orientations," of the shipping cases used are up, down, and on each of the four sides.

Step 2: The ASTM D4169 Truck Level 2 method is performed on the shipping cases in upright positions for three hours.

Step 3: The ASTM D4728 Truck method is performed with shipping cases at the six orientations for thirty minutes for each orientation.

Step 4: The shipping cases are dropped once at each of the six orientations for a total of six times.

After submitting the shipping cases to simulated shipping conditions, MRI images are taken for each personal care article by the MRI method at 25°C.

The MRI images prior to and after simulated shipping conditions are visually compared and graded of the shipping stability. The MRI images are compared on the amount of phase mixing, the presence of a zone of high concentration of hydrophobic benefit material, the orientation of the concentration of hydrophobic benefit material medial to the dispensing orifice, the amount of void volume and an orientation of the void volume at the end proximal to the dispensing orifice. If after submitting the shipping cases to simulated shipping conditions, the MRI images that show an excessive amount of mixing, the absence of a zone of high concentration of hydrophobic benefit material, an excessive amount of void volume and/or the volume is located medial or distal to the dispensing orifice; the personal care article would fail the dynamic stability shipping method. Conversely, if after submitting the shipping cases to simulated shipping conditions, the MRI images that show only a slight amount of mixing, the presence of a zone of high concentration of hydrophobic benefit material which is located medial to the dispensing orifice, a small amount of void volume located proximal to the dispensing orifice; the personal care article would pass the dynamic stability shipping method.

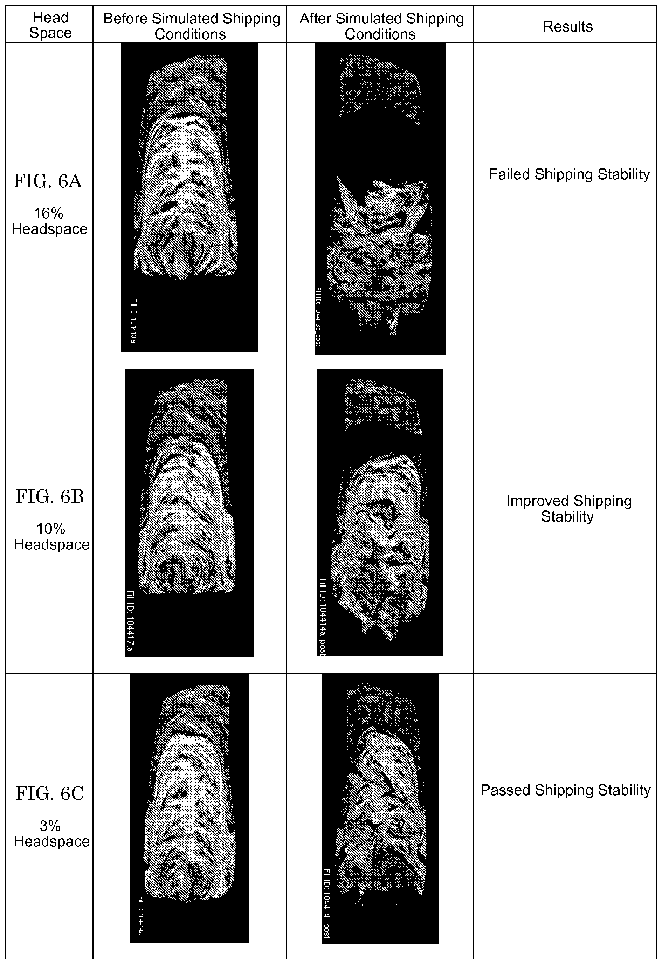

The results of the dynamic shipping stability method are shown below in FIG. 6A, FIG. 6B and FIG. 6C. As shown in FIG. 6A, the packages with 16% headspace shows extensive co-mixing of the two phases and thus, failed the shipping dynamic shipping stability method. As shown in FIG. 6B, the packages with 10% headspace shows improved dynamic shipping stability method as the zone of high concentration of hydrophobic benefit material is still apparent in the MRI image. As shown in FIG. 6C, the packages with 3% headspace shows the best shipping stability as the variable concentrations of hydrophobic benefit material is maintained after shipping protocol.

Ultracentrifugation Method:

The Ultracentrifugation Method is used to determine the percent of a structured domain or an opaque structured domain that is present in a personal care composition that comprises a surfactant phase or a surfactant component. The method involves the separation of a composition by ultracentrifugation into separate but distinguishable layers. The first personal care composition, second personal care composition and third personal care composition have multiple distinguishable layers, for example a non-structured surfactant layer, a structured surfactant layer, and a benefit layer.

First, dispense about 4 grams of personal care composition into Beckman Centrifuge Tube (11x60mm). Next, place the centrifuge tubes in an Ultracentrifuge (Beckman Model L8-M or equivalent) and ultracentrifuge using the following conditions: 50,000rpm, 18 hours, and 25°C.

After ultracentrifuging for 18 hours, determine the relative phase volume by measuring the height of each layer visually using an Electronic Digital Caliper (within 0.01mm). First, the total height is measured as $H_a$ which includes all materials in the ultracentrifuge tube. Second, the height of the benefit layer is measured as $H_b$. Third, the structured surfactant layer is measured as $H_c$. The benefit layer is determined by its low moisture content (less than 10% water as measured by Karl Fischer Titration). It generally presents at the top of the centrifuge tube. The total surfactant layer height ($H_s$) can be calculated by this equation:

$$H_s = H_a - H_b$$

The structured surfactant layer components may comprise several layers or a single layer. Upon ultracentrifugation, there is generally an isotropic layer at the bottom or next to the bottom of the ultracentrifuge tube. This clear isotropic layer typically represents the non-structured micellar surfactant layer. The layers above the isotropic phase generally comprise higher surfactant concentration with higher ordered structures (such as liquid crystals). These structured layers are sometimes opaque to naked eyes, or translucent, or clear. There is generally a distinct phase boundary between the structured layer and the non-structured isotropic layer. The physical nature of the structured surfactant layers can be determined through microscopy under polarized light. The structured surfactant layers typically exhibit distinctive texture under polarized light. Another method for characterizing the structured surfactant layer is to use X-ray diffraction technique. Structured surfactant layer display multiple lines that are often associated primarily with the long spacings of the liquid crystal structure. There may be several structured layers present, so that $H_c$ is the sum of the individual structured layers. If a coacervate phase or any type of polymer-surfactant phase is present, it is considered a structured phase.

Finally, the structured domain volume ratio is calculated as follows:

$$\text{Structured Domain Volume Ratio} = H_c / H_s * 100\%$$

If there is no benefit phase present, use the total height as the surfactant layer height, $H_s = H_a$.

Yield Stress and Zero Shear Viscosity Method:

The Yield Stress and Zero Shear Viscosity of a composition contained within a zone, can be measured either prior to combining the phases in a composition, or after combining the phases in a composition by separating the phases by suitable physical separation means, such as centrifugation, pipetting, cutting away mechanically, rinsing, filtering, or other separation means. In the case of testing from a product package, two zones can be selected from the package that contains at least two compositions that contain separate hydrophobic benefit material concentrations. In order to separate the zones, the product can be frozen at a temperature of at least -20°C for a period of at least 24 hours. The zones are then cut using a cutting implement such as a bandsaw. The cut portions are collected separately and allowed equilibrate to ambient conditions.

A controlled stress rheometer, such as a TA Instruments AR2000 Rheometer, is used to determine the Yield Stress and Zero Shear Viscosity. The determination is performed at 25°C with the 4 cm diameter parallel plate measuring system and a 1 mm gap. The geometry has a shear stress factor of 79580 m$^{-3}$ to convert torque obtained to stress. Serrated plates can be used to obtain consistent results when slip occurs.

First a sample of the composition is obtained and placed in position on the rheometer base plate, the measurement geometry (upper plate) moving into position 1 mm above the base plate. Excess phase at the geometry edge is removed by scraping after locking the geometry. If the phase comprises particles discernible to the eye or by feel (beads, e.g.) which are larger than about 150 microns in number average diameter, the gap setting between the base plate and upper plate is increased to the smaller of 4 mm or 8-fold the diameter of the 95$^{th}$ volume percentile particle diameter. If a phase has any particle larger than 5 mm in any dimension, the particles are removed prior to the measurement.

The determination is performed via the programmed application of a continuous shear stress ramp from 0.1 Pa to 1,000 Pa over a time interval of 4 minutes using a logarithmic progression, i.e., measurement points evenly spaced on a logarithmic scale. Thirty (30) measurement points per decade of stress increase are obtained. Stress, strain and viscosity are recorded. If the measurement result is incomplete, for example if material flows from the gap, results obtained are evaluated and incomplete data points excluded. The Yield Stress is determined as follows. Stress (Pa) and strain (unitless) data are transformed by taking their logarithms (base 10). Log(stress) is graphed vs. log(strain) for only the data obtained between a stress of 0.2 Pa and 2.0 Pa, about 30 points. If the viscosity at a stress of 1 Pa is less than 500 Pa-sec but greater than 75 Pa-sec, then log(stress) is graphed vs. log(strain) for only the data between 0.2 Pa and 1.0 Pa, and the following mathematical procedure is followed. If the viscosity at a stress of 1 Pa is less than 75 Pa-sec, the zero shear viscosity is the median of the 4 highest viscosity values (i.e., individual points) obtained in the test, the yield stress is zero, and the following mathematical procedure is not used. The mathematical procedure is as follows. A straight line least squares regression is performed on the results using the logarithmically transformed data in the indicated stress region, an equation being obtained of the form:

$$(1) \quad \mathrm{Log(strain) = m * Log(stress) + b}$$

Using the regression obtained, for each stress value (i.e., individual point) in the determination between 0.1 and 1,000 Pa, a predicted value of log(strain) is obtained using the coefficients m and b obtained, and the actual stress, using Equation (1). From the predicted log(strain), a predicted strain at each stress is obtained by taking the antilog (i.e., 10$^x$ for each x). The predicted strain is compared to the actual strain at each measurement point to obtain a %variation at each point, using Equation (2).

$$(2) \quad \mathrm{\%variation = 100 * (measured\ strain - predicted\ strain)/measured\ strain}$$

The Yield Stress is the first stress (Pa) at which %variation exceeds 10% and subsequent (higher) stresses result in even greater variation than 10% due to the onset of flow or deformation of the structure. The Zero Shear Viscosity is obtained by taking a first median value of viscosity in Pascal-seconds (Pa-sec) for viscosity data obtained between and including 0.1 Pa and the Yield Stress. After taking the first median viscosity, all viscosity values greater than 5-fold the first median value and less than 0.2x the median value are excluded, and a second median viscosity value is obtained of the same viscosity data, excluding the indicated data points. The second median viscosity so obtained is the Zero Shear Viscosity.

METHOD OF MANUFACTURE

In one embodiment, the personal care articles of the present invention are manufactured by a dual phase filler. The dual phase filler is associated with storage vessels, a combiner, a blender and nozzle for filling multiple personal care compositions. An example of a dual phase filler and associated software is manufactured by Antonio Mengibar Packaging Machinery of Barcelona, Spain. The surfactant phase and benefit phase of the personal care compositions are stored in separate storage vessel; each vessel equipped with a pump and a hose assembly. A programmed filler profile of the dual-phase filler controls the pumping of specific ratios of the two phases of the personal care compositions which result in the zones within a package. The two phases of the personal care compositions are pumped from the storage tanks into a combiner where the two phases are combined. After the phases are combined; they are mixed in a blender. From the blender, the resultant product is pumped via a hose into a single nozzle. The nozzle is placed into a container and fills a product package with a single resulting product. In some embodiments, the resultant product exhibits a distinct pattern of the phases which are visually distinct. In other embodiments, the resultant product exhibits a uniform appearance without a pattern. If a pattern is present, the pattern is selected from the group consisting of striped, marbled, geometric, and mixtures thereof.

In another embodiment, the personal care compositions of the present invention are manufactured according to the method disclosed in U.S. Patent Application No. 10/837,214 Publication No. 2004/0219119 A1 entitled "Visually distinctive multiple liquid phase compositions" filed by Wei et al. on April 30, 2004, published on November 18, 2004. Alternatively, it may be effective to combine toothpaste-tube filling technology with a spinning stage design. In still another embodiment, the personal care compositions are prepared by the method and apparatus as disclosed in U.S. Patent No. 6,213,166 issued to Thibiant et al. on April 10, 2001. The method and apparatus allow two or more compositions to be filled with a spiral configuration into a single product package. The method requires that at least two nozzles be employed to fill the compositions into a package. The package is placed on a moving stage and spun as the composition is introduced into the package.

Non-limiting examples of the personal care compositions, ratios of phases and filler profiles are disclosed in the examples below.

EXAMPLES

Table 1 shows non-limiting examples of the personal care products of the present invention and a comparative example. These personal care products are made and filled in a single chamber package. The personal care compositions of the present invention comprise various concentrations of hydrophobic benefit material through out the package. These personal care compositions of the present invention are filled in a package within multiples zones. The comparative example comprises uniform concentration of hydrophobic benefit material through out the package.

| Table 2: Examples of the Present Invention and Comparative Example | | | |
|---|---|---|---|
| | Inventive Example A | Inventive Example B | Comparative Example C |
| Surfactant Phase Composition | | | |
| Sodium Lauroamphoacetate [1.] | 4.9 | 4.9 | 4.9 |
| Sodium Trideceth Sulfate [2.] | 8.4 | 8.4 | 8.4 |
| Sodium Lauryl Sulfate | 8.4 | 8.4 | 8.4 |
| Trideceth-3 [3.] | 2.0 | 2.0 | 2.0 |
| Sodium Chloride | 4.75 | 4.75 | 4.75 |
| Guar hydroxypropyltrimonium chloride [4.] | 0.6 | 0.6 | 0.6 |
| Polyethyleneoxide[5.] | 0.15 | 0.15 | 0.15 |
| Xanthan gum[6.] | 0.2 | 0.2 | 0.2 |
| Hollow microspheres [7.] | 0.36 | 0.3 | 0.3 |
| Methyl chloro isothiazolinone and methyl isothiazolinone[8.] | 0.0005 | 0.0005 | 0.0005 |
| EDTA[9.] | 0.15 | 0.15 | 0.15 |

(continued)

| Table 2: Examples of the Present Invention and Comparative Example | | | |
|---|---|---|---|
| | Inventive Example A | Inventive Example B | Comparative Example C |
| Surfactant Phase Composition | | | |
| Sodium Benzoate | 0.2 | 0.2 | 0.2 |
| Citric Acid, titrate | pH=5.7 $\pm$ 0.2 | pH = 5.7 $\pm$ 0.2 | pH = 5.7 $\pm$ 0.2 |
| Perfume | 1.3 | 1.3 | 1.3 |
| Water | Q.S. | Q.S. | Q.S. |
| Benefit Phase Composition | | | |
| Petrolatum[10] | 70 | 70 | 70 |
| Mineral Oil[11.] | 30 | 30 | 30 |
| Filler Profile | Inventive Profile A | Inventive Profile B | Comparative Profile C |
| [1.] available from Cognis Chemical Corp. [2.] sulfanated to >95% sulfate from ICONOL® TDA-3 available from BASF Corp., [3.] ICONOL® TDA-3 available from BASF Corp., [4.] N-HANCE® 3196 Polymer from Aqualon of Wilmington, DE, [5.] POLYOX™ WSR-301 available from DOW® Chemical Corp., [6.] KELTRO™ 1000 available from CP Kelco, [7.] EXPANCEL® microspheres available from 091 WE40 d24, Akzo Nobel, [8.] KATHON® CG available for Rohm & Haas, [9.] DISSOLVINE® NA 2x available from Akzo Nobel, [10.] G2218 petrolatum from Sonneborn, [11.] HYDROBRTTE® 1000 White Mineral Oil available from Sonneborn. | | | |

The compositions described above can be prepared by conventional formulation and mixing techniques. The surfactant phase composition is made by first preparing a citric acid premix and then a polymer pre-mix. The citric acid premix is prepared by adding citric acid into water at a ratio of 1:3. The polymer premix is prepared by adding polyethyleneoxide and xanthan gum into trideceth-3. The following ingredients are then added into the main mixing vessel in the following sequence with agitation: water, guar hydroxypropyltrimonium chloride, hollow microspheres, sodium lauroamphoacetate, sodium trideceth sulfate, sodium lauryl sulfate, sodium chloride, sodium benzoate, and disodium EDTA. The citric acid premix is added into the main mixing vessel and the pH of the composition is adjusted to 5.7 $\pm$ 0.2. The polymer premix is next added into the main mixing vessel with continuous agitation. Perfume and methyl chloro isothiazolinone and methyl isothiazolinone are added while continuing the agitation until the composition is homogeneous. The resultant surfactant phase composition is fed into the dual-phase filler through a hose-assembly.

The benefit phase composition is prepared by first adding petrolatum into a mixing vessel. The mixing vessel has been heated to 82.2°C. Mineral oil is added into the mixing vessel with agitation. The benefit phase composition is cooled to 44°C through a scraped-wall heat-exchanger, such as that manufactured by Waukesha Cherry-Burrell, Delavan, WI. After cooling, the resultant benefit phase composition is fed into the dual-phase filler through a second hose-assembly.

The filler profiles A, B and C are examples of filling programs that specify the ratios of the surfactant and benefit phases within packages filled by a dual phase filler. Filler profiles A and B specify variable hydrophobic benefit material concentrations throughout the zones of the personal care articles of the present invention. Whereas filler profile C specifies uniform hydrophobic benefit material concentrations in the resultant personal care product within the package.

| Table 3: Dual Phase Filler Profiles for Example A and B | | | | | | | |
|---|---|---|---|---|---|---|---|
| Filler Profile A | | | | Filler Profile B | | | |
| Step | Dose (mL) | Benefit Material % | Surfactant % | Step | Dose (mL) | Benefit Material % | Surfactant % |
| 1 | 33.6 | 24 | 76 | 1 | 33.2 | 30 | 70 |
| 2 | 61.4 | 42 | 58 | 2 | 61.7 | 50 | 50 |
| 3 | 70.6 | 52 | 48 | 3 | 71.2 | 60 | 40 |
| 4 | 104.2 | 63 | 37 | 4 | 104.4 | 70 | 30 |
| 5 | 133.2 | 63 | 37 | 5 | 132.9 | 60 | 40 |

(continued)

| Table 3: Dual Phase Filler Profiles for Example A and B | | | | | | | |
|---|---|---|---|---|---|---|---|
| Filler Profile A | | | | Filler Profile B | | | |
| Step | Dose (mL) | Benefit Material % | Surfactant % | Step | Dose (mL) | Benefit Material % | Surfactant % |
| 6 | 155.2 | 52 | 48 | 6 | 154.7 | 50 | 50 |
| 7 | 174.9 | 42 | 58 | 7 | 194.6 | 40 | 60 |
| 8 | 194.5 | 32 | 68 | 8 | 223.1 | 20 | 80 |
| 9 | 223.5 | 15 | 85 | 9 | 248.7 | 10 | 90 |
| 10 | 249.0 | 7 | 93 | 10 | 280.0 | 20 | 80 |
| 11 | 280.2 | 15 | 85 | 11 | 289.5 | 30 | 70 |
| 12 | 289.5 | 27 | 73 | --- | --- | --- | --- |

| Table 4: Dual Phase Filler Profiles for Example C | | | |
|---|---|---|---|
| Filler Profile C | | | |
| Step | Dose (mL) | Benefit Material % | Surfactant % |
| 1 | 11 | 45 | 55 |
| 2 | 20 | 45 | 55 |
| 3 | 35 | 45 | 55 |
| 4 | 52 | 45 | 55 |
| 5 | 75 | 45 | 55 |
| 6 | 108 | 45 | 55 |
| 7 | 148 | 45 | 55 |
| 8 | 188 | 45 | 55 |
| 9 | 229 | 45 | 55 |
| 10 | 265 | 45 | 55 |
| 11 | 280 | 45 | 55 |

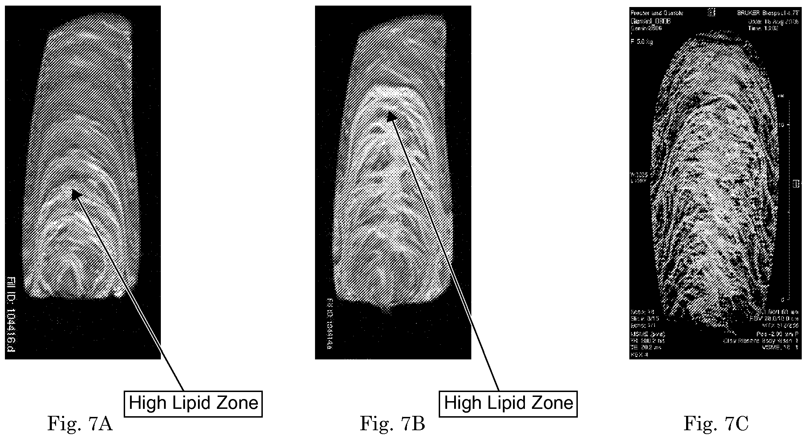

FIG. 7 depicts MRI images that illustrate the surfactant and hydrophobic benefit material distribution in a package of examples A, B and C. These images were taken by the MRI Method, described in detail in the Test Methods above. As shown in FIG.7, the comparative example C shows a uniform hydrophobic benefit material distribution throughout the package. Inventive examples A and B, in FIG. 7 show a variable hydrophobic benefit material distribution profile with higher benefit zones are highlighted with arrows.

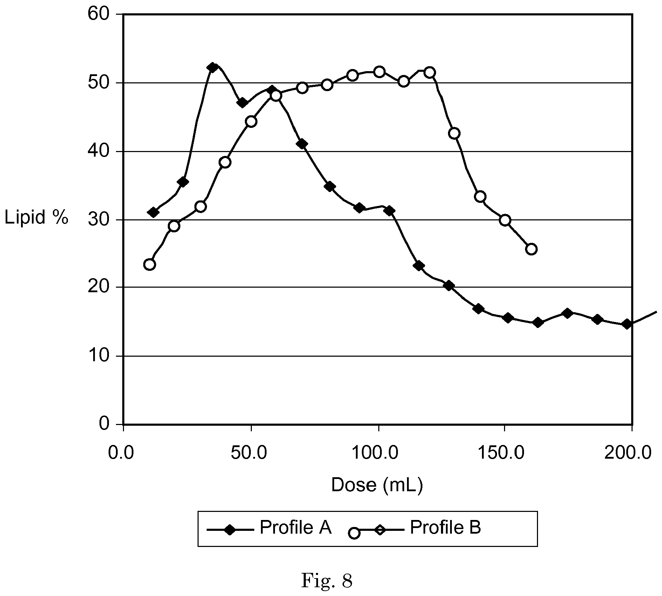

FIG. 8 is a chart of the hydrophobic benefit material distribution in examples A and B of the present invention. The Micro centrifugation Method, described in detail in the Test Methods above, was used to quantify the hydrophobic benefit material distribution in the inventive examples A and B. Profile A and profile B, shown in FIG. 8 clearly show a variable benefit distribution from the beginning, middle, and end of the dispensing.

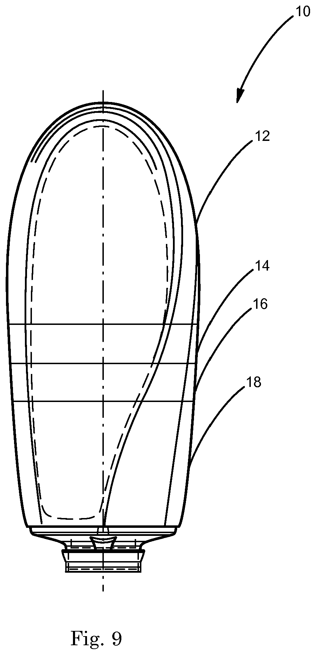

Referring to FIG. 9, a package 10 suitable for receiving a personal care composition is shown. The package 10 comprises a plurality of sections 12, 14, 16, and 18 that are separable from each other. As described further hereafter, the package 10 can be used in a quality assurance method to assess the profiles of the hydrophobic benefit material and/or a surfactant benefit material (or other materials) after a filling operation. In one embodiment the package 10 is part of a quality assurance check in a high speed manufacturing line, wherein after a filling operation the sections 12, 14, 16, and 18 of package 10 are separated and the personal care composition in each section is separately analyzed to determine the amount/volume of hydrophobic benefit material and/or surfactant material by weight of the personal care composition in the separated section. The analysis of the benefit materials can be performed using any of the methods (or others) described herein. The centrifuge and/or MRI methods described herein may be used in one embodiment. These material profiles can be combined to yield an overall material profile curve for the package 10, similar to that shown in FIG. 8, of the hydrophobic benefit material and/or surfactant benefit material which can be compared to a manufacturing specification for the product.

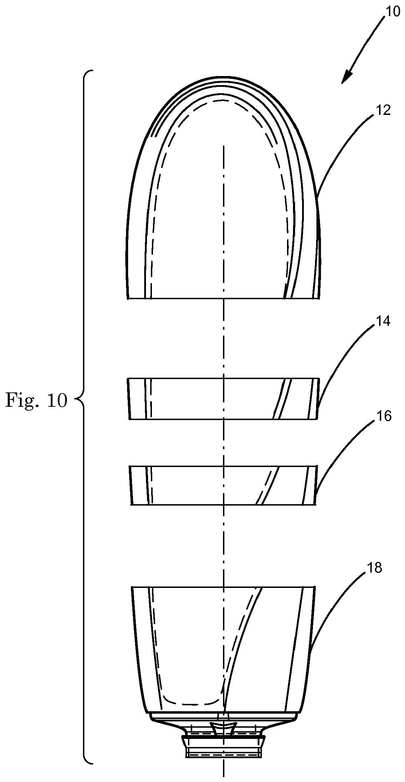

The sections 12, 14, 16, and 18 can be held together by one or more fasteners (not shown), such as a screw or bolt, as known in the art. In one embodiment, two screws extend from section 12 through to section 18 thereby interconnecting each of sections 12, 14, 16, and 18. When the two screws are removed, each of the section 12, 14, 16, and 18 can be fully separated from each other as shown in FIG. 10. Alternatively, a separate fastener could be employed between each section, although this would necessitate removal of more than 2 fasteners to separate all of the sections 12, 14, 16, and 18. While four sections 12, 14, 16, and 18 are shown, it will be appreciated that more (or fewer) sections can be provided depending upon the desired resolution of the overall material profile curve for the package 10. For example, increasing the number of separable sections should provide a higher resolution because more data points are generated for the profile curve of the package. In one embodiment, the package 10 comprises between 2 and 8 sections and in another embodiment the package 10 comprises between 4 and 6 sections.

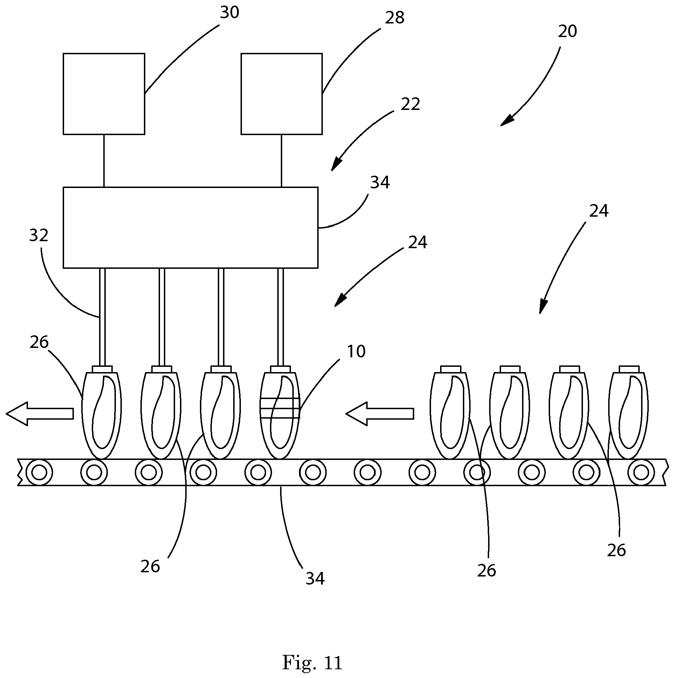

Referring to FIG. 11, a high speed manufacturing line 20 is shown schematically. The manufacturing line 20 comprises a filling station 22 that simultaneously receives a plurality of containers 24 comprising a plurality of consumer packages 26 and one or more packages 10. The consumer packages 26 are non-separable and intended for eventual distribution to an end user or consumer after filling with a personal care composition, such as a rinse-off body wash. As shown in FIG. 11, the package 10 can be provided to the filling station 22 intermittently with the plurality of containers 24.

In one embodiment, the filling station 22 comprises two tanks 28 and 30. Tank 28 contains a first composition comprising the hydrophobic benefit material, and tank 30 contains a second composition comprising one or more surfactants. The first and second compositions can be mixed as discussed previously. While two tanks are shown, it will be appreciated that more tanks and compositions can be provided as desired. The tanks 28 and 30 are connected by hose assemblies to a filler 34 comprising a plurality of nozzles 32. The first and second compositions are pumped from the tanks 28 and 30 to the filler 34. There is a nozzle for filling each of the plurality of containers 24. Each nozzle is configured so that the first and second compositions are dispensed simultaneously into each container according to a predetermined ratio that can vary along the length of the container, an exemplary predetermined ratio being described in Table 3. The first and second compositions thereby form the personal care composition that is dispensed from the container during use. The filling station 22 can be configured so that the filler 34, along with the plurality of nozzles 32, can be displaced vertically toward and away from the plurality of containers 24 during a filling operation so an appropriate profile of the first and second compositions can be achieved along the length of a container.

After a filling operation is complete, the plurality of filled containers 24 are moved away from the filling station 22 by a conveyor 34, and a new set of unfilled containers are moved into position within the filling station 24. As shown in FIG. 11, the new plurality of containers 24 may omit the separable package 10. The package 10 is removed from the manufacturing line 20 after filling.

After removal of the package 10 from the manufacturing line 20, the sections 12, 14, 16, and 18 are separated from each other along with the dosage of the personal care composition disposed within each section. In the context of a personal care compositions that is a rinse-off type body wash, the viscosity is typically such that the dosages of the personal care composition disposed within each section of the package 10 tends to stay within its section after separation of the sections from each other. The personal care composition within each section of package 10 can then be removed from its section of package 10 and individually analyzed to determine the amount of hydrophobic benefit material that section of the package 10. The Micro-Centrifugation Method described previously may be used to determine the amount of hydrophobic material present in the one or more sections of the package 10, although other methods may also be employed in combination with or as substitute for the Micro-Centrifugation Method. For example, the MRI Method might be used in combination with the Micro-Centrifugation method, wherein the package 10 is imaged by an MRI machine before separating the sections of the package to obtain the dosages of the personal care composition for micro-centrifuge testing.

Once the dosage of the personal care composition has been separated/dispensed from its section and analyzed, a curve can be plotted that represents the variation of the concentration of the hydrophobic material (or some other material) along the length of the package 10 by weight of either the dosage of the personal care composition within a section of the package 10 or by overall weight of the personal care composition within the entire package 10. This curve can be compared to a known calibration curve to determine whether the manufacturing process is within limits or if corrective action is necessary in order to assure that the containers are filled according to the desired profile.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm." The citation of any document is not to be construed as an admission that it is prior art with respect to the present invention. To the extent that any meaning or definition of a term in this document conflicts with any meaning or definition of the same term in a cited document, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1.  A method for dispensing a personal care composition, comprising:

    dispensing a plurality of doses of the personal care composition from a package and applying each dose to human skin, wherein the personal care composition comprises a surfactant and a lipid;
    dispensing a first dose of the plurality of doses from the package, wherein the first dose has a concentration of the lipid by weight of the first dose;
    dispensing a second dose of the plurality of doses from the package after the first dose, wherein the second dose has a concentration of the lipid by weight of the second dose that is greater than the concentration of the lipid by weight of the first dose; and
    dispensing a third dose of the plurality of doses from the package after the second dose, wherein the third dose has a concentration of the lipid by weight of the third dose that is less than the concentration of the lipid by weight of the second dose.

2.  The method according to claim 1, wherein each dose of the personal care composition is topically applied to the skin and then rinsed from the skin.

3.  The method according to any of the preceding claims, wherein the lipid is petrolatum.

4.  The method according to any of the preceding claims, wherein personal care composition is a body wash and the doses are applied to a leg of a user.

5.  The method according to any of the preceding claims, further comprising dispensing a fourth dose of the personal care composition, a fifth dose of the personal care composition, and a sixth dose of the personal care composition.

6.  The method according to any of the preceding claims, wherein the concentration of the lipids of the first dose is greater than 30% by weight of the first dose.

## Patentansprüche

1.  Verfahren zur Ausgabe einer Körperpflegezusammensetzung, umfassend:

    Ausgeben einer Vielzahl von Dosen der Körperpflegezusammensetzung aus einer Einkammerpackung und Anwenden jeder Dosis auf der menschlichen Haut, wobei die Körperpflegezusammensetzung ein Tensid und ein Lipid umfasst;
    Ausgeben einer ersten Dosis von einer Vielzahl von Dosen aus der Verpackung, wobei die erste Dosis eine Lipidkonzentration abhängig vom Gewicht der ersten Dosis aufweist;
    Ausgeben einer zweiten Dosis der Vielzahl von Dosen aus der Verpackung nach der ersten Dosis, wobei die zweite Dosis eine Lipidkonzentration abhängig vom Gewicht der zweiten Dosis aufweist, die größer als die Lipidkonzentration abhängig vom Gewicht der ersten Dosis ist; und
    Ausgeben einer dritten Dosis der Vielzahl von Dosen aus der Verpackung nach der zweiten Dose, wobei die dritte Dose eine Lipidkonzentration abhängig vom Gewicht der dritten Dosis aufweist, die geringer als die Lipidkonzentration abhängig vom Gewicht der zweiten Dosis ist.

2.  Verfahren nach Anspruch 1, wobei jede Dosis der Körperpflegezusammensetzung topisch auf der Haut angewendet wird und dann von der Haut abgespült wird.

3.  Verfahren nach einem der vorstehenden Ansprüche, wobei das Lipid Petrolatum ist.

**4.** Verfahren nach einem der vorstehenden Ansprüche, wobei die Körperpflegezusammensetzung ein Körperwaschmittel ist und die Dosen auf einem Bein eines Benutzers angewendet werden.

**5.** Verfahren nach einem der vorstehenden Ansprüche, ferner umfassend die Ausgabe einer vierten Dosis der Körperpflegezusammensetzung, einer fünften Dosis der Körperpflegezusammensetzung und einer sechsten Dosis der Körperptlegezusammensetzung.

**6.** Verfahren nach einem der vorstehenden Ansprüche, wobei die Konzentration der Lipide der ersten Dosis größer als 30 Gew.-% der ersten Dosis ist.

**Revendications**

**1.** Procédé de distribution d'une composition de soin personnel, comprenant :

la distribution d'une pluralité de doses de la composition de soin personnel à partir d'un conditionnement à chambre unique et l'application de chaque dose sur la peau humaine, dans lequel la composition de soin personnel comprend un agent tensioactif et un lipide ;
la distribution d'une première dose parmi la pluralité de doses à partir du conditionnement, dans lequel la première dose a une concentration du lipide en poids de la première dose ;
la distribution d'une deuxième dose parmi la pluralité de doses à partir du conditionnement après la première dose, dans lequel la deuxième dose a une concentration du lipide en poids de la deuxième dose qui est supérieure à la concentration du lipide en poids de la première dose ; et
la distribution d'une troisième dose parmi la pluralité de doses à partir du conditionnement après la deuxième dose, dans lequel la troisième dose a une concentration du lipide en poids de la troisième dose qui est inférieure à la concentration du lipide en poids de la deuxième dose.

**2.** Procédé selon la revendication 1, dans lequel chaque dose de la composition de soin personnel est appliquée localement sur la peau, puis rincée de la peau.

**3.** Procédé selon l'une quelconque des revendications précédentes, dans lequel le lipide est de la vaseline.

**4.** Procédé selon l'une quelconque des revendications précédentes, dans lequel la composition de soin personnel est un produit de lavage pour le corps et les doses sont appliquées sur une jambe d'un utilisateur.

**5.** Procédé selon l'une quelconque des revendications précédentes, comprenant en outre la distribution d'une quatrième dose de la composition de soin personnel, d'une cinquième dose de la composition de soin personnel, et d'une sixième dose de la composition de soin personnel.

**6.** Procédé selon l'une quelconque des revendications précédentes, dans lequel la concentration des lipides de la première dose est supérieure à 30 % en poids de la première dose.

Fig. 1A

Fig. 1B

Fig. 2A

Fig. 2B

Fig. 2C

Fig. 3

EP 2 391 545 B1

Fig. 4B

Fig. 4A

EP 2 391 545 B1

Along Height

Fig. 5A

Fig. 5B

| Head Space | Before Simulated Shipping Conditions | After Simulated Shipping Conditions | Results |
|---|---|---|---|
| **FIG. 6A**<br><br>16% Headspace | | | Failed Shipping Stability |
| **FIG. 6B**<br><br>10% Headspace | | | Improved Shipping Stability |
| **FIG. 6C**<br><br>3% Headspace | | | Passed Shipping Stability |

High Lipid Zone

Fig. 7A

High Lipid Zone

Fig. 7B

Fig. 7C

Fig. 8

Fig. 9

Fig. 10

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0243321 A
- US 06744305 A, McCall
- US 3929678 A, Laughlin
- US 60680149 B
- US 2658072 A
- US 2438091 A
- US 2528378 A
- US 20040092415 A1
- US 5487884 A, Bisset
- US 5681852 A
- US 5652228 A, Bisset
- US 6395691 B, Liang Sheng Tsaur
- US 6645511 B, Aronson
- US 6759376 B, Zhang
- US 6780826 B, Zhang
- WO 20060182699 A, Taylor
- US 837214 A
- US 20040219119 A1
- US 6213166 B, Thibiant

### Non-patent literature cited in the description

- *Vaughan in Cosmetics and Toiletries,* vol. 103
- **C. D. VAUGHAN.** Solubility, Effects in Product, Package, Penetration and Preservation. *Cosmetics and Toiletries,* October 1988, vol. 103
- **MCCUTCHEON'S.** Detergents and Emulsifiers. allured Publishing Corporation, 1986
- **MCCUTCHEON'S.** Functional Materials. 1992
- CTFA Cosmetic Ingredient Handbook. The Cosmetic, Toiletries, and Fragrance Association, Inc, 1992, vol. 1988